# EUROPEAN PATENT APPLICATION

(11) **EP 3 888 666 A1**
(43) Date of publication of application: **06.10.2021**
(21) Application number: 20168012.1
(22) Date of filing: 03.04.2020
(51) Int. Cl.: A61K 35/28, A61P 15/08, C12N 5/0775

(54) **COMPOSITION**

(71) Applicant: InoCells B.V., 6167 RD Geleen (NL)
(72) Inventor: Mehdi, Ahmad Akhondi Mohammad, 6167 RD Geleen (NL); Jafar, Ai, 6167 RD Geleen (NL); Somayeh, Ebrahimi Barough, 6167 RD Geleen (NL)
(74) Representative: Renkema, Jaap

(57) **Abstract**

The invention relates to a composition comprising endometrial mesenchymal stem cells (EnMSC) for use in a method for the treatment of diminished ovarian response and a method for making a composition comprising endometrial mesenchymal stem cells.

## Description

### FIELD OF THE INVENTION

The present invention relates to a composition for use in a method for the treatment of diminished ovarian response (DOR) and a method for making such a composition comprising endometrial mesenchymal stem cells.

### STATE OF THE ART

Due to the delay in child-bearing age in the modern society, many female subjects are experiencing infertility, for example as the result of diminishing ovarian reserve during aging, e.g. infertile female subjects above 40 years of age. Diminished ovarian response (DOR, also called decreased ovarian reserve or diminished ovarian reserve) is a condition suffered by female subjects over the age of 40, which is further characterised by for example decreased anti-mullerian hormone (AMH), increased follicle-stimulating hormone (FSH), a decreased antral follicle count (AFC) and a low oocytes quality compared to healthy female subjects of that age. Further DOR subjects have regular menses and reduced capacity of the ovaries to produce oocytes. The oocytes produced by a female subject with DOR are typically of poorer quality as compared to those produced by females with good ovarian reserve. The development of DOR is linked to the process of follicular depletion and decline in oocyte quality.

DOR subjects have the desire for biological offspring. Currently, the only method of achieving pregnancy in these subjects is by means of Assisted Reproductive Techniques (ART), such as *in vitro* fertilisation (IVF), which require ovarian stimulation with high doses of gonadotropins. Various modalities have been tried to improve the outcome in subjects with DOR undergoing assisted reproductive technology. These include high-dose FSH treatment, luteinising hormone (LH) supplementation, gonadotropin-releasing hormone (GnRH) antagonist cycle, and use of adjuvant treatments such as estradiol priming, growth hormone, L-arginine and dehydroepiandrosterone (DHEA). These gonadotropin treatments and fertility treatments have negative side effects such as abdominal pain, nausea, vomiting, weight gain, acne, breast pain or tenderness and mood swings, which can lead to discontinued treatment. Further, although gonadotropin treatments are widely used to promote the development of early antral follicles to the preovulatory stage, many DOR subjects do not respond to the gonadotropin therapy. Female subjects with DOR may have fewer numbers of oocytes during oocyte retrieval; hence, fewer embryos for transfer and fewer chances of conception when compared with a female subject having normal ovarian reserve. These DOR subjects may need cancellation of the IVF cycle midway either due to the absence of follicular development, due to lack of oocytes retrieved, lack of successful fertilisation or increased pregnancy failure (e.g. high miscarriage rate, which is thought to be due to the initial low oocyte quality found in DOR subjects). This leads many DOR subjects to either rely on donor oocyte programs or adoption programs.

Sills et al. [1] discloses a successful *in vitro* fertilisation of a premature ovarian failure (POF) subject with an oocyte donated from a twin sister. This non-autologous IVF cell treatment leads to non-biological offspring. POF has an age of onset before the age of 40 years, typically before the age of 35 years. Although POF is related to infertility, DOR and POF are distinct medical conditions, they have different causes and there is no evidence for DOR being a precursor to POF.
WO 2016/140910 discloses a pre-treatment with AMH prior to controlled ovarian hyperstimulation (COH; further referred to ovarian stimulation) or superovulation treatments, such as gonadotropin treatments, for DOR subjects. The disclosed method is said to increase the yield of oocytes induced by COH/superovulation which can then be used for subsequent IVF treatment of DOR subjects. This method may lead to biological offspring if oocyte retrieval and pregnancy are successful, but does not abrogate the negative side effect of the IVF or gonadotropin treatment nor the reduced oocyte quality and increased pregnancy failure.
It is a challenge for infertility experts to manage a subject with DOR, and most subjects have the desire for biological offspring. Thus, there is a demand for DOR treatments that have at least one of the following advantages fewer negative side effects, allow for autologous oocytes, new oocyte formation, increased oocyte quality, non-IVF conception, allow for natural conception, re-establishing lower FSH serum levels compared to the DOR state (towards normal serum levels), re-establishing higher AMH serum levels compared to the DOR state (towards normal serum levels) and re-establishing higher estradiol (E2) serum levels compared to DOR state (towards normal serum levels). Serum levels are understood to mean blood serum levels.

### SUMMARY OF THE INVENTION

The present invention provides a composition comprising endometrial mesenchymal stem cells for use in a method for the treatment of diminished ovarian response.

It further relates to a method for making a composition comprising endometrial mesenchymal stem cells.

It was surprisingly found that the composition according to the invention generates germ cell-like cells and/or re-establishes certain hormone serum levels to non-DOR serum levels (towards normal serum levels), such as for example at least one of the following serum levels AMH, FSH and estradiol.

The composition of the invention has at least one of the following advantages: fewer negative side effects, allows for autologous oocytes, new oocyte formation, increased oocyte quality, non-IVF conception, allows for natural conception, regeneration of ovarian function, oogenesis, re-establishing lower FSH serum levels compared to the DOR state (towards normal serum levels), re-establishing higher AMH serum levels compared to the DOR state (towards normal serum levels) and re-establishing higher estradiol (E2) serum levels compared to DOR state (towards normal serum levels).

### DETAILED DESCRIPTION OF THE INVENTION

The present invention provides a composition comprising endometrial mesenchymal stem cells for use in a method for the treatment of diminished ovarian response.

The composition comprises endometrial mesenchymal stem cells that are preferably autologous and human.

### Definitions

The term 'markers' or 'biomarkers' (also known as 'biological markers' or 'molecular markers' or 'genetic markers' or 'protein markers' and the like) as used herein refers to a measurable indicator of some biological state or condition or diseases or complications relating to a disease. Biomarkers can be any molecule (inorganic or organic molecule, protein or nucleotide sequence) or substances circulating in the blood, or present in the serum, biofluid or tissue, or genes or polynucleotides expressed in specific tissues or cells. In an embodiment of the present invention, the markers are proteins and/or hormones present or expressed in the human subject's or subject's blood serum or the endometrial cells or stem cells from a human subject or subject.

The term 'estradiol' (also known as 'estrogen' or 'oestradiol' or 'E2' or '17β-estradiol' and the like) as used herein refers to a measurable indicator of some biological state.

The term 'passage' (also known as 'cell passage' and the like) as used herein refers to subcultures. A passage number is the number of times a cell culture has been subcultured. 'Flow-cytometry-passage' is different from the term 'passage' or 'cell passage' herein refers to the number of passages through the flow-cytometer.

### DOR

A DOR subject is a female subject over 40 years of age having regular menses whose response to ovarian stimulation or fecundity is reduced compared with female subjects of comparable age.

DOR is distinct from menopause or premature ovarian failure (POF, also referred to as primary ovarian insufficiency (POI)), as defined by Practice Committee of American Society for Reproductive Medicine 2015 [2]. Female subjects diagnosed with POI/POF are under 40 years of age and have postmenopausal FSH serum levels and 3-6 months without menses (e.g. secondary amenorrhea or oligo-menorrhea). In POI/POF subjects FSH serum levels are above 40 mlU/mL on at least two different occasions, AMH serum levels are below 1 ng/mL and the AFC is less than 3.

DOR is characterised by FSH serum levels at above 10 mIU/mL, AMH serum levels are below 2 ng/mL and the AFC is less than 5-7. DOR can further be diagnosed through high FSH and/or high estradiol serum levels measured in the early follicular phase (such as day 2-3 at the start of the menstrual cycle) during a clomiphene challenge test and/or reduced ovarian volume.

Basal FSH serum levels may be a good predictor of the size of the remaining follicle pool. Elevated basal serum FSH levels are indicative of DOR, and female subjects with increased basal FSH serum levels frequently have decreased oocytes retrieved in IVF programs. Basal serum levels of FSH and LH of day 2-3 of the menstrual cycle are preferably used to test for ovarian reserve screening. Day 1 of the menstrual cycle is understood to be the first day of the menstruation. The first half of the menstrual cycle is the follicular phase followed by the second half, which is the luteal phase, both phases last approximately 14 days in an average menstrual cycle of 28 days. The luteal phase is also referred to as the 'secretory phase'. FSH serum levels should be measured on various occasions to rule out discontinuous ovarian activity as a cause of increased gonadotropins. However, only increased FSH serum levels is of limited utility as far as assessment of ovarian reserve is concerned for the management of infertility. Most estradiol in a healthy and non-DOR subject is produced by the granulosa cells of the ovaries by the aromatization of androstenedione (produced in the theca folliculi cells) to estrone, followed by conversion of estrone to estradiol through 17β-hydroxysteroid dehydrogenase.

Ovarian dysfunction recovery, such as in for example DOR treatment, may be evaluated using the serum E2 level in animal and human subjects over a determined time interval, such as for example 1 week, 6 weeks, 1 month, 3 months and 6 months, using a statistical comparison between serum E2 level after treatment and before treatment.

An increased basal serum E2 level after administration of the composition according to the invention may be considered as a positive sign of treatment efficiency in addition to at least one of the following decreased FSH serum levels, increased AMH serum levels and increased antral follicle count.

The amount of estradiol (E2) in blood serum of animal and human subjects may be measured using an enzyme-linked immunosorbent assay (ELIZA) technique.

AMH is preferably another marker for diminished ovarian reserve. AMH, in a healthy and non-DOR subject, is secreted by cells in the developing egg sacs (follicles in the ovaries). AMH serum levels between 2-6 ng/mL are considered normal. Serum AMH levels below 2 ng/mL may be further indicative of DOR, more preferably are serum levels of less than 0.5-1.1 ng/mL.

### EnMSC

The endometrium regrows from a 1-2 mm thickness after menstrual shedding to 14 mm thickness in the secretory phase (i.e. luteal phase) of the menstrual cycle and is able to completely regenerate after parturition, and in postmenopausal female subjects when exposed to estradiol replacement therapy. Even after extensive iatrogenic destructive procedures such as ablation, the endometrium regrows in some female subjects who continue to bleed (in about 25-75 % of female subjects).

The endometrium consists of two layers, the functionalis layer and basalis layer. The functionalis layer includes the upper two thirds of the glands surrounded by loose vascularised stroma. Being a germinal supplier for new functionalis layer replacement in each menstrual cycle the basalis layer is composed of the lower one thirds of glands, stroma and large vessels. The functionalis layer sheds monthly with menstrual blood arising from changes in hormone levels and is quickly reconstructed after menstruation. This huge regenerative ability suggests that the endometrium has a stem cell basis that supports the tissue maintenance/regrowth. Endometrial stem cells (EnSC) were initially thought to be located only in the basalis layer. New evidence has shown that there are some stem cells in the functionalis layer of the endometrium.

EnSCs have been identified based on properties such as clonogenicity, long-term culturing capability, multilineage differentiation potential and expression of stem cell markers. Three kinds of endometrial stem cells exist in the endometrium: epithelial progenitor cells, endometrial mesenchymal stem cells (EnMSCs) and endothelial stem cells. The epithelial progenitor cell population is located within the residual glands of the basalis layer. The subpopulation of endometrial stem cells that express CD146 and CD140b/PDGFRb are preferably endometrial mesenchymal stem cells. They are mainly located near small vessels in the functionalis and basalis layers.

Epithelial progenitor cells cannot be obtained from menstrual blood, as they are not present in menstrual blood. Epithelial progenitor cells are characterised by being positive for SSEA-1 and LGR5 as markers. The epithelial progenitor cells release growth factors such as FGF2 and EGF. Growth factors, such as FGF2 and EGF, impact the proliferation of other cells co-isolated from the endometrial tissue sample. The endometrial tissue sample preferably includes at least the basalis layer, and most preferably the basalis and functionalis layers. The endometrial tissue sample is preferably a fresh tissue sample from the secretory phase of the subject's menstrual cycle. A fresh tissue sample from secretory phase of the subject's menstrual cycle is thought to significantly improve the composition. Endometrial tissue sample can be obtained in sterile conditions. The endometrial tissue sample and derived stem cells are preferably low in contaminants compared to menstrual blood and its derived stem cells, which are contaminated with vaginal microorganisms. The endometrial tissue sample has preferably a lower percentage of necrotic cells than menstrual blood sample. The endometrial tissue sample preferably comprises epithelial progenitor cells, endometrial mesenchymal stem cells (EnMSCs) and endothelial stem cells. The endometrial mesenchymal stem cells have a higher clonogenicity than menstrual stem cells. The epithelial progenitor cells have a high proliferation rate and telomerase activity and are preferably supporting the EnSCs in the endometrial tissue sample and the EnMSCs derived from the endometrial tissue sample. Methods for quantifying the proliferation rate and telomerase activity are for example real time PCR for telomere markers, such as hTERT, and KI67 staining for proliferation, as referred to in [3]. The endometrial tissue derived EnMSCs may also show an increased hTERT level, thus increased telomerase activity. Methods of endometrial tissue sample acquisition are known in the art.

The EnMSCs and EnSCs used in the invention are preferably endometrium tissue derived endometrial stem cells, rather than menstrual blood derived menstrual stem cells. EnSCs derived from endometrium tissue, compared to menstrual stem cells, may have a higher telomerase activity, lower necrotic cells percentage, low contamination and/or higher clonogenicity. Menstrual stem cells are positive for SOX2 and CD117 markers. Further, EnSCs may be characterised by at least the absence of SOX2 and CD117 markers (negative) and/or by at least the presence of SUSD2, W5C5 and LGR5 markers (positive).

The EnSCs may be additionally to the SUSD2, W5C5 and LGR5 markers characterised by being positive for SSEA4 and hTERT markers.

The EnSCs may be further characterised by being positive for CD73, CD105, CD90, CD29, CD146, CD166, STRO1, LGR5 (EnSCs), SSEA-4 (EnSCs), h-TERT, SUSD2, N-cadherin and Nanog markers and negative for SOX2 and CD117 markers.

The EnMSCs or composition may be further characterised by the being positive for CD146 or CD146 and PDGFRb markers.

The EnMSCs or composition may be further characterised in that said endometrial mesenchymal stem cells express Oct-4, CD146 and STRO-1.

The EnMSCs or composition may be positive for CD90, CD146 and CD105 markers and negative for CD34 and CD31 markers.

The EnMSCs may be further characterised by at least the absence of SOX2 and CD117 markers (negative) and/or by at least the presence of SUSD2, W5C5 and LGR5 markers (positive).

The EnMSCs may be further characterised by being positive for SSEA4 and hTERT markers.

EnMSCs are easily accessible, low cost, have minimal ethical hurdle, low immunogenicity and/or low tumorgenicity. EnMSCs differentiation ability is preferably higher than that of other stem cells. The EnMSCs may for example differentiate *in vitro* into chondrogenic, adipogenic and osteogenic lineages better than other mesenchymal stem cells.

The EnMSCs may be further characterised by one or more of the following plastic adherence, being fibroblast like, having multi-lineage differentiation potential, expression of classical mesenchymal stem cell surface markers and stable karyotype in culture.

EnMSCs were surprisingly found to generate germ cell-like cells and/or re-establish certain hormone serum levels to non-DOR serum levels (towards normal serum levels), such as for example AMH, FSH and estradiol.

The EnMSCs are preferably autologous. Autologous is understood to mean from the DOR-subject. Autologous stem-cell transplantation is a transplantation of stem cells that are removed from a subject and transplanted back into the same subject, with optional banking between the removal and the transplantation.

### Process

The invention further relates to a method for making a composition comprising endometrial mesenchymal stem cells, comprising the steps of:
a. Submersing an endometrial tissue sample in a balanced salt solution;
b. Washing of the endometrial tissue sample, obtained from step a., with a buffered saline solution;
c. Mincing the endometrial tissue sample, obtained from step b., to produce a minced endometrial tissue sample;
d. Digesting the minced endometrial tissue sample, obtained from step c., to produce a digestate, comprising epithelial cells and stem cells;
e. Centrifuging the digestate, obtained from step d.;
f. Separating through filtration the digestate, obtained from step e., into an epithelial cells fraction and a stem cells fraction;
g. Culturing the endometrial mesenchymal stem cells from step f. in a medium;
h. Characterisation of endometrial mesenchymal stem cells through flow cytometry, wherein said endometrial mesenchymal stem cells are positive for CD90, CD146 and CD105 markers and negative for CD34 and CD31 markers;
i. Characterisation of endometrial mesenchymal stem cells through multipotency property, such as differentiation into osteocytes and adipocyte cells.

The method for making a composition comprising endometrial mesenchymal stem cells preferably comprises autologous endometrial mesenchymal stem cells.

Step a. of the method for making a composition comprises submersing an endometrial tissue sample in a balanced salt solution, for example as a transferring media.

The endometrial tissue sample is preferably a fresh tissue sample from secretory phase of the subject's menstrual cycle.

The balanced salt solution is preferably Hanks fluid, which comprises 0.5-4 wt% penicillin, 0.5-4 wt% streptomycin and 0.25-1.5 wt% amphotericin, preferably 1.75-2.25 wt% penicillin, 1.75-2.25 wt% streptomycin and 0.75-1.25 wt% amphotericin. Alternatives for the Hanks fluid may be Dulbecco's Modified Eagle Medium: Nutrient Mixture F12 (DMEM/F12) (for 24 hours after obtaining the endometrial tissue sample) or PBS (for 4-6 hours after obtaining the endometrial tissue sample.

Step b. of the method for making a composition comprises washing of the endometrial tissue sample, obtained from step a., with a buffered saline solution.

The buffered saline solution is preferably PBS buffered phosphate saline which comprises 0.5-4 wt% penicillin, 0.5-4 wt% streptomycin and 0.25-1.5 wt% amphotericin, preferably 1.75-2.25 wt% penicillin, 1.75-2.25 wt% streptomycin and 0.75-1.25 wt% amphotericin. Alternatives for the PBS buffered phosphate saline can be sterile saline (for example NaCl 0.9 wt%) or cell culture mediums such as DMEM/F12. Step c. of the method for making a composition comprises mincing the endometrial tissue sample, obtained from step b., to produce a minced endometrial tissue sample. The endometrial tissue sample in step c. may be minced using for example scalpels, razors or scissors to produce a minced endometrial tissue sample.

Step d. of the method for making a composition comprises digesting the endometrial tissue sample, obtained from step c., to produce a digestate, comprising epithelial cells and stem cells.

The digesting in step d. may be conducted with proteolytic collagenase 1, such as for example collagenase A. The digesting with collagenase 1, such as for example collagenase A, may be for 30-120 minutes, preferably 35-90 minutes, more preferably 45-75 minutes, most preferably 46-60 minutes.

The digesting in step d. may be conducted in PBS buffered phosphate saline which comprises 0.5-4 wt% penicillin, 0.5-4 wt% streptomycin and 0.25-1.5 wt% amphotericin, preferably 1.75-2.25 wt% penicillin, 1.75-2.25 wt% streptomycin and 0.75-1.25 wt% amphotericin. Alternatives for the PBS buffered phosphate saline can be sterile saline (for example NaCl 0.9 wt%).

Collagenase 1, such as for example collagenase A, may be obtained from cultures free of animal-derived materials. Collagenase preparations contain the activity of several proteases, including collagenase, caseinase, clostripain, and trypsin. Collagenase A, contains levels of proteolytic activity similar to type 1 and type 2 collagenases.

Step e. of the method for making a composition comprises centrifuging the digestate, obtained from step d.. The digestate in step e. may be centrifuged at room temperature (e.g. 20 °C) for 1-15 minutes, preferably for 2-10 minutes, most preferably for 4-8 minutes at 500-5000 rpm, preferably 800-1500, most preferably 1000-1300. Any suitable centrifuge can be used, for example a Hettich Universal 320 Centrifuge. The medium used in the centrifugation step may be DMEM/F12 with 10 wt% FBS.

Step f. of the method for making a composition comprises separating through filtration the digestate into an epithelial cells fraction and a stem cells fraction.

The filtration may comprise filtration of the digestate with 70 µm and 40 µm cell strainers. An example of cell strainers used according to the invention may be the BD Sterile Cell Strainer 40 micron (BD 352340) and the BD Sterile Cell Strainer 70 micron (BD 352350).

Step f. may be followed by a substep f2. comprising the centrifugation of the obtained a stem cells fraction at for example 1200-1500 rpm for 5 minutes at room temperature. The first cell strainer of 70 µm may be used to separate out the cell debris and undigested tissue and the second cell strainer of 40 µm maybe used to separate out epithelial cells. Step g. of the method for making a composition comprises culturing the endometrial mesenchymal stem cells from step f. in a medium.

The culturing the endometrial mesenchymal stem cells in step g. may be in an incubator at 35-38 °C, 2-10 % CO₂ and 95 % humidity of air for 2 weeks, preferably at 37 °C, 5 % CO₂ and 95 % humidity of air for 2 weeks, most preferably at 37 °C, in a medium, 5 % CO₂ and 95 % humidity of air for 2 weeks.

The method for making a composition comprising endometrial mesenchymal stem cells, wherein in step g. the medium is preferably exchanged every 3 days.

The medium in the culturing step g. may comprise DMEM/F12, 8-12 wt% FBS and 0.25-1.5 wt% penicillin, 0.25-1.5 wt% streptomycin, preferably 0.75-1.25 wt% penicillin, 0.75-1.25 wt% streptomycin. The medium in the culturing step g. preferably inhibits or is not suitable for blood and endothelial cells to grow in.

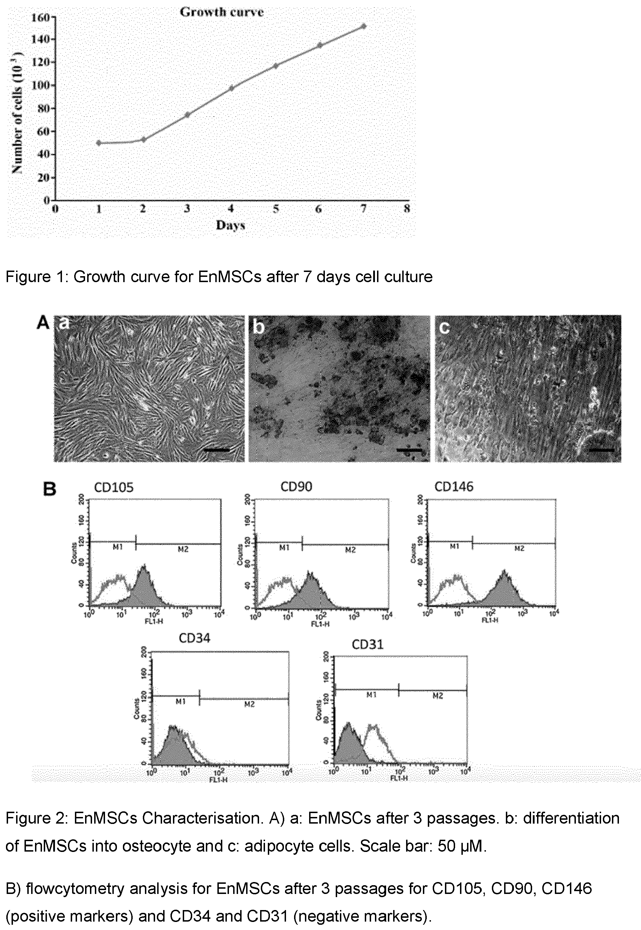

The cell culture doubling time of the culture may be 49.9 hours. An example of a growth curve is shown in figure 1.

The endometrial mesenchymal stem cells may be passaged 1-8 times, preferably 2-5, most preferably 3 times before being cell characterised and administered. After a certain number of passages, for example 3, the endometrial mesenchymal stem cells may have over-grown the side population cells, endothelial cells or non-proliferative cells. For example, in and after passage 3 the cell culture is homogenous.

Step h. of the method for making a composition comprises characterising endometrial mesenchymal stem cells from the cultured stem cells fraction, through flow cytometry, wherein said endometrial mesenchymal stem cells are positive for CD90, CD146 and CD105 markers and negative for CD34 and CD31 markers.

The characterisation of step h. may be conducted using one flow-cytometry-passage through flow cytometry. The characterisation can be conducted using a flow cytometer. In step h. the cells may be in Hanks fluid with 10 wt% fetal bovine serum (FBS), 0.25-1.5 wt% penicillin, 0.25-1.5 wt% streptomycin and 0.25-1.5 wt% amphotericin, preferably 0.75-1.25 wt% penicillin, 0.75-1.25 wt% streptomycin and 0.75-1.25 wt% amphotericin.

Step h. may be followed by a substep h2. comprising the centrifugation at 1200-1500 rpm for 5 minutes at room temperature.

Step i. of the method for making a composition comprises characterisation of endometrial mesenchymal stem cells through multipotency property, such as differentiation into osteocytes and adipocyte cells.

Characterisation of endometrial mesenchymal stem cells through multipotency property, such as differentiation into osteocyte cells may be confirmed by alizarin red s staining. Alizarin red s is an anthraquinone dye used to stain for calcium deposits, which are indicators of mature osteocytes.

Characterisation of endometrial mesenchymal stem cells through multipotency property, such as differentiation into adipocyte cells may be confirmed by oil red o staining. Oil red O is a dye that strongly stains lipids.

A further preferred method for making a composition comprising endometrial mesenchymal stem cells, comprising the steps of:
a. Submersing the endometrial tissue sample in Hanks fluid;
b. Washing the endometrial tissue sample, obtained from step a., with PBS buffered phosphate saline comprising penicillin, amphotericin and streptomycin;
c. Mincing the endometrial tissue sample, obtained from step b., to produce a minced endometrial tissue sample;
d. Digesting the endometrial tissue sample, obtained from step b., with proteolytic collagenase to produce a digestate, comprising epithelial cells and stem cells;
e. Centrifuging the digestate, obtained from step d.;
f. Separating through filtration the digestate into an epithelial cells fraction and a stem cells fraction using a 70 µm and 40 µm cell strainers;
g. Culturing the endometrial mesenchymal stem cells from step f. in an incubator at 37 °C, in a medium, 5 % CO₂ and 95 % humidity of air for 2 weeks;
h. Characterisation of endometrial mesenchymal stem cells through flow cytometry, wherein said endometrial mesenchymal stem cells are positive for CD90, CD146 and CD105 markers and negative for CD34 and CD31 markers;
i. Characterisation of endometrial mesenchymal stem cells through multipotency property, such as differentiation into osteocytes and adipocyte cells.

### Formulation

The composition comprising endometrial mesenchymal stem cells for use in a method for the treatment of diminished ovarian response may have a pH between 7.2-7.4. The composition comprising endometrial mesenchymal stem cells for use in a method for the treatment of diminished ovarian response may comprise a physiologically relevant solution selected from the following PBS solution, autologous serum, sterile normal saline (for example NaCl 0.9 wt%) or cell culture mediums, such as DMEM/F12 as used in culturing, preferably at a pH of 7.2-7.4; preferably the solution is a PBS solution at a pH of 7.2-7.4.

An example of a physiologically relevant PBS solution is an aqueous solution comprising 137 mmol/L NaCl, 2.7 mmol/L KCI, 10 mmol/L Na₂HPO₄ and 1.8 mmol/L KH₂PO₄ at a pH of 7.4.

The composition according to the invention may comprise endometrial mesenchymal stem cells at a concentration of 0.5-10 million cell number/mL, preferably 0.95-8 million cell number/mL, most preferably 1.5-6.5 million cell number/mL.

The composition according to the invention for use as a medicament may be defined by its effect of increasing the serum levels of AMH above 1 ng/mL, increasing the mature follicles count to 2 follicles in each ovary, estradiol (E2) serum levels to above 30 pg/mL, at least 5-7 units decrease in FSH serum levels, increasing ovarian volume to 6-10 cm³ or increasing the antral follicle count to 3-9 up to 6 months after transplantation.

### Administration of Composition

The composition according to the invention may be administered freshly or after being frozen, preferably the composition is administered freshly. When administration occurs after cryo-freezing re-culturing after the thawing process is preferred. During re-culturing the cells may reach to log phase of growth and can then be administered. The composition according to the invention may be administered to the artery, the ovarian artery, intra-ovarianly or to at least one ovary. When administered intra-ovarianly cells are preferably injected through the vagina using for example ultrasonic guidance.

The composition according to the invention is preferably administered in 1 to 5 doses, preferably 2 to 4 doses, at an amount of 0.5-2 million cell number per dose, preferably 0.8-1.3 million cell number per dose.

### Banking

The invention further relates to a method for banking of endometrial mesenchymal stem cell or composition according to the invention.

The invention further relates to a method for banking of endometrial mesenchymal stem cell or composition according to the invention, comprising:
a. Obtaining endometrial mesenchymal stem cells from a subject;
b. Checking for bacterial, yeast, or fungal contamination under a microscope;
c. Testing a sample of endometrial mesenchymal stem cells for mycoplasma using Gibco's MycoTect kit (Cat. No. 15672-017);
d. After the endometrial mesenchymal stem cells have reached late log phase, resuspend the endometrial mesenchymal stem cells in freeze medium at 5 000 000 - 20 000 000 cells/mL);
e. Centrifuging the resuspended endometrial mesenchymal stem cells in 50 mL Falcon tube at 1000 g for 15 minutes;
f. Suctioning away supernatant from centrifuged endometrial mesenchymal stem cells, add freeze medium and triturate cells until homogeneous;
g. Aliquoting 1 mL of the endometrial mesenchymal stem cells obtained from step f. in to vials and freeze vials at -20 °C freezer in a container for 3 hours;
h. Transferring the container to a - 80 °C freezer and store overnight.
i. Storing the next day, the vials obtained from step f. in a rack in a liquid N₂ tank.

The composition according to the invention may further comprise optional components different from the previously mentioned components of the composition, such as additives, wherein the total of the previously mentioned components and the optional components is 100 wt% of the total composition. Accordingly, the invention relates to a composition consisting of the previously mentioned components and the optional components.

It is noted that the invention relates to all possible combinations of features described herein, preferred in particular are those combinations of features that are present in the claims. It will therefore be appreciated that all combinations of features relating to the composition according to the invention; all combinations of features relating to the process according to the invention and all combinations of features relating to the composition according to the invention and features relating to the process according to the invention are described herein.

It is further noted that the terms "including", "comprising", "having", "containing" or "involving" do not exclude the presence of other elements. However, it is also to be understood that a description on a product/composition comprising certain components also discloses a product/composition consisting of these components. The product/composition consisting of these components may be advantageous in that it offers a simpler, more economical process for the preparation of the product/composition. Similarly, it is also to be understood that a description on a process comprising certain steps also discloses a process consisting of these steps. The process consisting of these steps may be advantageous in that it offers a simpler, more economical process.

When values are mentioned for a lower limit and an upper limit for a parameter, ranges made by the combinations of the values of the lower limit and the values of the upper limit are also understood to be disclosed.

The invention is now elucidated by way of the following examples, without however being limited thereto.

### FIGURE DESCRIPTION

Figure 1: Growth curve for EnMSCs after 7 days cell culture
Figure 2: Accordingly figure 2 shows (A) Morphological characteristics and *in vitro* differentiation of human EnMSCs into mesenchymal lineages. (a) Human EnMSCs (passage 3) have differentiated into (b) mineralising cells stained with Alizarin Red S. (c) Adipocytes stained with Oil Red O (scale bar: 50 µm). (B) Flow cytometry analysis on the isolated human EnMSCs for mesenchymal stem cell (CD90, CD105), endometrial stem cell (CD146), hematopoietic (CD34), and endothelial (CD31) markers. Blue lines indicate background fluorescence obtained with isotype control IgG1 for CD31 and IgG2a for CD105, CD90, CD146 and CD34.

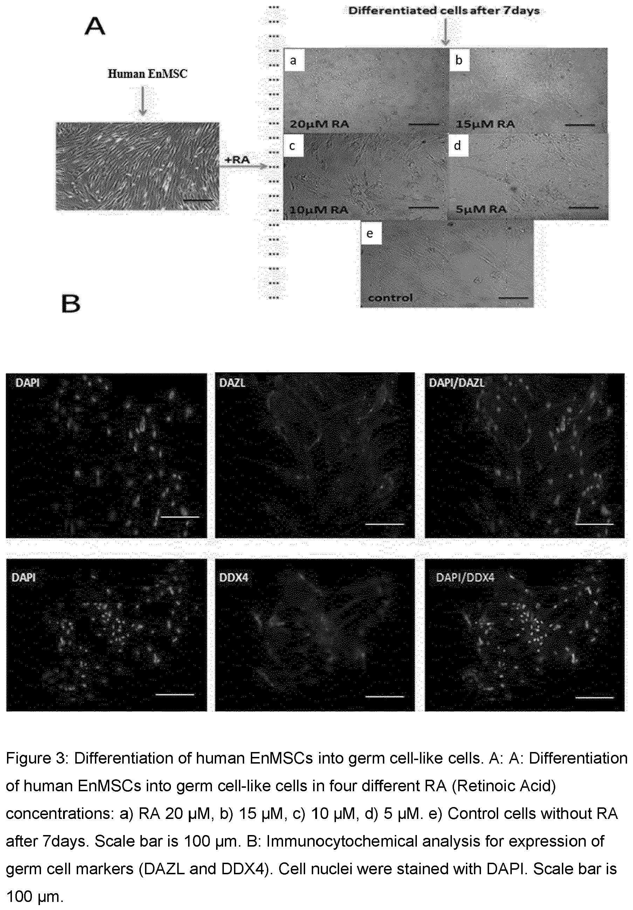

Figure 3: Accordingly figure 3 shows A: Differentiation of human EnMSCs into germ cell-like cells in four different retinoic acid (RA) concentrations: a-human EnMSCs after 3 days/ RA 20 µM, b-Differentiated cells after 7days/ RA15 µM, c-Differentiated cells after 7 days /RA 10µM, d-Differentiated cells after 7days/ RA 5µM, e-Control cells after 7 days/ without RA B: Immunocytochemical analysis for germ cell expression markers in 2D medium 7 days after 10 µm RA induction. Cell nuclei were stained with DAPI (scale bar is 100 µm).

### EXAMPLES

### EnMSC

A human endometrial tissue sample from a non-DOR subject was collected in the luteal phase. The in Hank's fluid submersed human endometrial tissue sample, comprising functionalis and basalis layers, was washed in Dulbecco's phosphate buffered saline (2 wt% penicillin, 2 wt% streptomycin and 1 wt% amphotericin), minced, and then digested in Hank's balanced salt solution (HBSS) containing collagenase 1, i.e. collagenase A, (1 mg/mL) for 30-45 minutes at 37 °C with agitation. The resultant digestate was then centrifuged in a Hettich Universal 320 Centrifuge. The resulting pellet was washed in phosphate buffered saline (PBS). The resultant centrifuged digestate, comprising epithelial cells and stem cells, was then passed through a 70 µM and a 40 µM strainer (BD Biosciences, USA, 93070) to remove glandular epithelial components. The endometrial stem cells (EnSCs) were cultured in DMEM/F12 medium with 10 wt% fetal bovine serum (FBS), 1 wt% antibiotic penicillin,), 1 wt% streptomycin, in plastic flasks (25 cm²) and then incubated at 37 °C in humidified chamber (5 % CO₂ and at 95 % humidity of air) and allowed to replicate to confluence (figure 1). A flow cytometry analysis was undertaken on the obtained human EnMSCs for mesenchymal stem cell (CD90, CD105), endometrial stem cell (CD146), hematopoietic (CD34), and endothelial (CD31) markers. To morphological characterise and show *in vitro* differentiation of human EnMSCs ((a) of figure 2A) into mesenchymal lineages the human EnMSCs (after 3 passages) were stained with Alizarin Red showing differentiation into mineralising cells ((b) of figure 2A) and were stained with Oil Red O showing differentiation into adipocytes ((c) of figure 2A).

Osteogenic differentiation: Endometrial mesenchymal stem cells were expanded and passaged in DMEM with 10% FBS. osteogenic differentiation was induced in the third passage cells by plating the EnMSCs at 2×10⁴ cells per cm², allowing the cells to reach confluence and then incubating for a further 24 hr. The media was then changed to differentiation media containing DMEM/F12 supplemented with 10% FBS with 10 mM β-glycerophosphate, 0.1 µM dexamethasone, and 200 µM ascorbic acid-2-phosphate. Differentiation medium was changed every 3-4 days for 21 days.

Alizarin red S staining: The cells were washed with PBS and fixed in 10% (v/v) formaldehyde. After 15 min, Alizarin red S 2% (pH 4.1) was added to each flask. The flasks were incubated at room temperature for 20 min and then they were washed four times with dH2O shaken for 5 min. The secretion of calcified ECM was observed as red nodules with Alizarin red S staining

Adipogenic differentiation: Cells derived from whole isolates of endometrium were expanded and passaged in DMEM with 10% FBS. Adipogenic differentiation was induced in the third passage cells by plating the EnMSCs at 2×10⁴ cells per cm², allowing the cells to reach confluence and then incubating for a further 24 hr. The media was then changed to differentiation media containing DMEM/F12 supplemented with 10% FBS with insulin (10 g/ml), dexamethasone (1 M), indomethacin (200 M) and isobutylmethylxanthine (0.5 mM). Differentiation medium was changed every 3-4 days for 21 days.

Oil Red O Staining: Oil Red O Stain was used to confirm the presence of lipid in differentiated cells. Cells were washed with PBS, fixed in 2% paraformaldehyde, 0.2% glutaraldehyde in PBS for 15 min and then rinsed with PBS. Then they were stained with Oil Red O (reconstituted in isopropanol) for 10 min and rinsed in 60% isopropanol followed by PBS. Lipid droplets were visualized in red under light microscopy.

### In vitro Model

Differentiation of human EnMSCs into germ cell-like cells for for example oogenesis/regeneration of ovarian function (e.g. development of oocytes and/or hormone secretion, such as estradiol and AMH).

The human endometrial mesenchymal stem cells were induced to differentiate through incubation in culture medium at 37 °C, in 95 % humidity of air and 5 % CO₂ for 7 days. The medium culture was DMEM (Invitrogen, USA) with 10 wt% FBS, 1 wt% penicillin, 1 wt% streptomycin and retinoic acid (RA) (Invitrogen, USA) in four different concentrations: 5, 10, 15 and 20 µM (figure B 3b-e). A control group was prepared in which the human EnMSCs were cultured in DMEM with no RA as a differentiation inducing factor for the same time as the other 4 samples (figure B 3f). The DMEM medium in the culture was changed every other day. After 7 days the cultured human EnMSCs were shown to differentiate into germ cell-like cells by immunofluorescence staining for DAPI, DAZL and DDX4 (figure 3A).

### In vivo Model

The composition in accordance with the invention was used in a method of treating DOR in a DOR rat model. Additionally, blank (i.e. + or positive) and negative controls were run.

Rats were randomly assigned to the following 3 groups:
- A DOR rat group (is called Stem cell transplanted group in table 1) (n=10). The DOR rat model was established by intraperitoneal injection with 200 mg/kg of cyclophosphamide (CTX) in to the rat on the first day and then 8 mg/kg/day for the 15 consecutive days. 2 weeks after the DOR rat model was established the DOR rats were injected in the tail intravenously with a composition comprising human EnSCs, comprising EnMSCs (100 *µ*L, at a concentration of 1×10⁶/mL) with a microinjector.
- A blank control group (is called control + in table 1) (n=10). These were non-DOR rats, i.e. normal, without any treatment administration 2 weeks after the DOR rat model was establishment of the DOR rat group.
- A negative control group of DOR rat model (is called control - in table 1) (n=10). The DOR rat model was established by intraperitoneal injection with 200 mg/kg of cyclophosphamide (CTX) in to the rat on the first day and then 8 mg/kg/day for the 15 consecutive days. No treatment was administered 2 weeks after the DOR rat model establishment of the DOR rat group.

1 week and 6 weeks after the treatment the serum FSH, estradiol (E2) and AMH serum levels were measured. The MSCs treatment led to a re-establishment of non-DOR, e.g. normal, serum levels of FSH, estradiol (E2) and AMH in DOR rats.

**Table 1: Serum FSH, estradiol (E2) and AMH serum levels in DOR treated rat model, compared to positive and negative controls.**

| FSH (IU/L) | 1week after treatment | ! 6weeks after treatment ! |
|---|---|---|
| Control + | 8.6 ± 1.04 | 8.7 ± 1.38 |
| Control - | 11.1± 1.84 | 12.3± 2.04 |
| Stem cell transplanted group | 9.8± 2.04 | 9.2± 0.54 |
| | | |

| 2 (ng/L) | 1week after treatment | 6weeks after treatment |
|---|---|---|
| Control + | 48.6± 4.042 | 55.4± 1.54 |
| Control - | 41± 2.67 | 45± 3.04 |
| Stem cell transplanted group | 52± 2.54 | 58± 2.76 |
| | | |

| AMH (pg/ml) | 1week after treatment | 6weeks after treatment |
|---|---|---|
| Control + | 170.2± 8.076 | 179.3± 5.46 |
| Control - | 114± 10.14 | 128± 3.56 |
| Stem cell transplanted group | 140± 6.78 | 145± 6.98 |

Treatment of DOR rats with a composition comprising EnMSCs led to a decrease in serum FSH serum levels in comparison to DOR rats without treatment. Treatment of DOR rats with the MSCs led to an increase in serum AMH and estradiol serum levels in comparison to DOR rats without treatment.

### Clinical Data

Female human subject (herein after called subjects) are recruited fulfilling the following inclusion criterion, females over 40 years of age, diagnosed with diminished ovarian reserve, a serum FSH level above 20 IU/L or an AMH serum level of less than 1 ng/mL, fertile husband (fertile sperm), normal karyotype, have an IVF failure history. Further the subjects did not have the following exclusion criterion, primary amenorrhea, abnormal karyotype (e.g. turner syndrome, fragile X syndrome), thyroid dysfunction, severe endometriosis, contraindications for pregnancy, prior personal history of ovarian cancer, prior personal history of breast cancer, history of serious drug allergy or allergic constitution, autoimmune disease, history of severe familial genetic disease, HIV+, hepatitis B+, hepatitis C+, mental disease, communicate obstruction and alcohol or other substance abuse.

A composition comprising autologous EnMSCs according to the invention is administered to the subject intra-ovarianly.

The subjects show at least one of the below improvements:
- Increased serum levels of AMH to above 1ng/mL (preferably up to 2.5 ng/mL) up to 6 months after administering the composition according to the invention (transplantation);
- Increased mature follicles count to 2 follicles in each ovary up to 6 months after transplantation;
- Increased estradiol (E2) serum levels to above 30 pg/mL (preferably up to 45 pg/mL) up to 6 months after transplantation;
- At least 5-7 units decrease in follicle-stimulating-hormone (FSH) serum levels up to 6 months after transplantation;
- Increased ovarian volume to 6-10 cm³ up to 6 months after transplantation;
- Increased antral follicle count to 3-9 up to 6 months after transplantation.

All of the mentioned data is collected during clinical trial, but the improvement in one of the mentioned criteria can show the positive ovary response to this medication.

### REFERENCES

[1] Reprod Biol Endocrinol. 2010 Mar 25;8:31. doi: 10.1186/1477-7827-8-31. IVF for premature ovarian failure: first reported births using oocytes donated from a twin sister. Sills ES, Brady AC, Omar AB, Walsh DJ, Salma U, Walsh AP.
[2] Fertil Steril. 2015 Mar;103(3):e9-e17. doi: 10.1016/j.fertnstert.2014.12.093. Epub 2015 Jan 10. Testing and interpreting measures of ovarian reserve: a committee opinion. Practice Committee of the American Society for Reproductive Medicine.
[3] Hum Reprod. 2013 Oct;28(10):2695-708. doi: 10.1093/humrep/det285. Epub 2013 Jul 11. SSEA-1 isolates human endometrial basal glandular epithelial cells: phenotypic and functional characterization and implications in the pathogenesis of endometriosis. Valentijn AJ1, Palial K, Al-Lamee H, Tempest N, Drury J, Von Zglinicki T, Saretzki G, Murray P, Gargett CE, Hapangama DK.

## Claims

1. A composition comprising endometrial mesenchymal stem cells for use in a method for the treatment of diminished ovarian response.

2. The composition according to claim 1, wherein said endometrial mesenchymal stem cells are human autologous endometrial mesenchymal stem cells.

3. The composition according to claim 1 or 2, wherein said endometrial mesenchymal stem cells are positive for CD90, CD146 and CD105 markers and negative for the CD34 and CD31 marker.

4. The composition according to anyone of claims 1-3, wherein said endometrial mesenchymal stem cells express Oct-4, CD146 and STRO-1.

5. The composition according to anyone of claims 1-4, wherein said endometrial mesenchymal stem cells are derived from an endometrial tissue sample.

6. The composition according to anyone of claims 1-5, wherein said composition is administered intra-ovarianly or to at least to one ovary.

7. The composition according to anyone of claims 1-6, wherein said composition further comprising a physiologically relevant solution selected from the following PBS solution, autologous serum, sterile normal saline or cell culture mediums at a pH of 7.2-7.4.

8. The composition according to anyone of claims 1-7, wherein said endometrial mesenchymal stem cells are at a concentration of 0.5-10 million cell number/mL, preferably 0.95-8 million cell number/mL, most preferably 1.5-6.5 million cell number/mL.

9. The composition according to anyone of claims 1-8, wherein said composition is administered in 1 to 5 doses, preferably 2 to 4 doses, at an amount of 0.5-2 million cell umber per dose, preferably 0.8-1.3 million cell umber per dose.

10. A method for making a composition comprising endometrial mesenchymal stem cells, comprising the steps of:
a. Submersing an endometrial tissue sample in a balanced salt solution;
b. Washing of the endometrial tissue sample, obtained from step a., with a buffered saline solution;
c. Mincing the endometrial tissue sample, obtained from step b., to produce a minced endometrial tissue sample;
d. Digesting the minced endometrial tissue sample, obtained from step c., to produce a digestate, comprising epithelial cells and stem cells;
e. Centrifuging the digestate, obtained from step d.;
f. Separating through filtration the digestate, obtained from step e., into an epithelial cells fraction and a stem cells fraction;
g. Culturing the endometrial mesenchymal stem cells from step f. in a medium;
h. Characterisation of endometrial mesenchymal stem cells through flow cytometry, wherein said endometrial mesenchymal stem cells are positive for CD90, CD146 and CD105 markers and negative for CD34 and CD31 markers;
i. Characterisation of endometrial mesenchymal stem cells through multipotency property, such as differentiation into osteocytes and adipocyte cells.

11. The method for making a composition comprising endometrial mesenchymal stem cells, according to claim 10, wherein said endometrial mesenchymal stem cells are autologous endometrial mesenchymal stem cells

12. The method for making a composition comprising endometrial mesenchymal stem cells, according to claim 11, wherein in step f. the endometrial mesenchymal stem cells are cultured at 35-38 °C, 2-10 % CO₂ and 95 % humidity of air for 2 weeks, preferably at 37 °C, 5 % CO₂ and 95 % humidity of air for 2 weeks.

13. The method for making a composition comprising endometrial mesenchymal stem cells, according to claim 11 or 12, wherein in step f. the medium is exchanged every 3 days.

14. The method for making a composition comprising endometrial mesenchymal stem cells, according to anyone of claims 10-13, comprising the steps of:
a. Submersing the endometrial tissue sample in Hanks fluid;
b. Washing the endometrial tissue sample, obtained from step a., with PBS buffered phosphate saline comprising penicillin, amphotericin and streptomycin;
c. mincing the endometrial tissue sample, obtained from step b., to produce a minced endometrial tissue sample;
d. Digesting the endometrial tissue sample, obtained from step b., with proteolytic collagenase to produce a digestate, comprising epithelial cells and stem cells;
e. Centrifuging the digestate, obtained from step d.;
f. Separating through filtration the digestate into an epithelial cells fraction and a stem cells fraction using a 70 µm and 40 µm cell strainers;
g. Culturing the endometrial mesenchymal stem cells from step f. in an incubator at 37 °C, in a medium, 5 % CO₂ and 95 % humidity of air for 2 weeks;
h. Characterisation of endometrial mesenchymal stem cells through flow cytometry, wherein said endometrial mesenchymal stem cells are positive for CD90, CD146 and CD105 markers and negative for CD34 and CD31 markers;
i. Characterisation of endometrial mesenchymal stem cells through multipotency property, such as differentiation into osteocytes and adipocyte cells.

15. A method of treating diminished ovarian response, comprising the step of administering a composition comprising endometrial mesenchymal stem cells, according to anyone of claims 1-9.
